# EUROPEAN PATENT SPECIFICATION

(11) **EP 3 629 942 B1**
(45) Date of publication and mention of the grant of the patent: **28.12.2022**
(21) Application number: 18878147.0
(22) Date of filing: 21.05.2018
(51) Int. Cl.: A61B 17/072, A61B 17/00

(54) **A CONTINUOUS STAPLING INSTRUMENT**
KONTINUIERLICHES KLAMMERINSTRUMENT
INSTRUMENT D'AGRAFAGE CONTINU

(30) Priority: 24.05.2017 TR 201707597
(43) Date of publication of application: 08.04.2020
(73) Proprietor: Senuysal, Hilal, Istanbul (TR); Tansug, Tugrul, 34740 Kadikoy/Istanbul (TR); Elitas, Meltem, Tuzla/Istanbul (TR)
(72) Inventor: Senuysal, Hilal, Istanbul (TR); Tansug, Tugrul, 34740 Kadikoy/Istanbul (TR); Elitas, Meltem, Tuzla/Istanbul (TR)
(74) Representative: Dericioglu, E. Korhan
(86) International application number: PCT/TR2018/050257
(87) International publication number: WO 2019/098954

(56) References cited:
- EP-A2- 2 583 630
- EP-A2- 2 792 311
- GB-A- 1 429 386
- US-A1- 2005 187 576
- US-A1- 2011 290 854
- US-A1- 2011 290 855

## Description

### Field of the Invention

The present invention relates to a continuous stapling instrument which performs stapling without moving the instrument away from the area where the operation is being performed during stapling of the tissue in open, closed and/or robotic surgeries.

### Background of the Invention

In the currently used surgical stapling instruments, cartridges of different lengths loaded with staples which are attached to the end of the instrument are removed after they are applied on the organ during open or closed surgical operations, and a new cartridge is inserted for a second stapling on the same or another organ. The stapling instrument is required to be removed from the surgical site for replacement of the cartridge. After the new cartridge is inserted, the instrument is directed to the target organ again.

United States patent document no. US7641095, an application known in the state of the art, discloses a surgical instrument which enables to perform stapling continuously. In the said device, a staple package is placed on the first loading portion. After stapling is completed, the second loading portion is wrapped around the roller.

United States patent document no. US3650453, an application in the state of the art, discloses a stapler cartridge system developed for being used in surgical operations. The staples in the said cartridge system move along the belt. It is disclosed that, when the said staple cartridge is inserted in the stapling instrument, continuous stapling will be performed.

United States patent document no. US7000819, an application in the state of the art, discloses a stapler firing mechanism developed for being used in surgical operations. There is provided a gear that can move towards a single direction. The said invention resembles the present invention in form in terms of the gear system, however does not disclose about the staple wires on a flexible strip (belt) being wrapped around the said gears.

EP2583630 A2 discloses a surgical stapler including an anvil assembly and a cartridge assembly. The anvil assembly defines staple forming depressions. One or both of the anvil assembly and the cartridge assembly are pivotable relative to the other between an open position and a clamped position. The cartridge assembly includes a first plurality of staples and a second plurality of staples. The first plurality of staples is initially positioned in alignment with the staple forming depressions of the anvil assembly for ejection from the cartridge assembly. The second plurality of staples is movably supported in the cartridge assembly from a first position misaligned with the staple forming depressions of the anvil assembly to a second position aligned with the staple forming depressions for subsequent ejection from the cartridge assembly.

GB1429386 discloses a surgical stapling instrument comprising a main body portion, mounting means at the forward end of said main body portion for rigidly holding a staple cartridge or for rigidly holding an adaptor for attachment to a staple cartridge, drive means housed in the main body portion for delivering power from the instrument to said cartridge for ejecting staples from said cartridge, said mounting means being freely rotatably connected to said main body portion to enable said cartridge when attached to the instrument to be freely rotated relative to the main body portion.

### Problems Solved by the Invention

The object of the present invention is to provide a continuous stapling instrument which enables to perform transection of organs or bowel anastomosis during the entire surgical operation without the need for removal and insertion of the staple belt during surgery, and thus enables the user to perform stapling with less effort. Another object of the present invention is to provide a continuous stapling instrument which enables a safer and shorter stapling without being removed from within the patient during the operations.

### Detailed Description of the Invention

A continuous stapling instrument developed to fulfill the objects of the present invention is illustrated in the accompanying figures, in which:

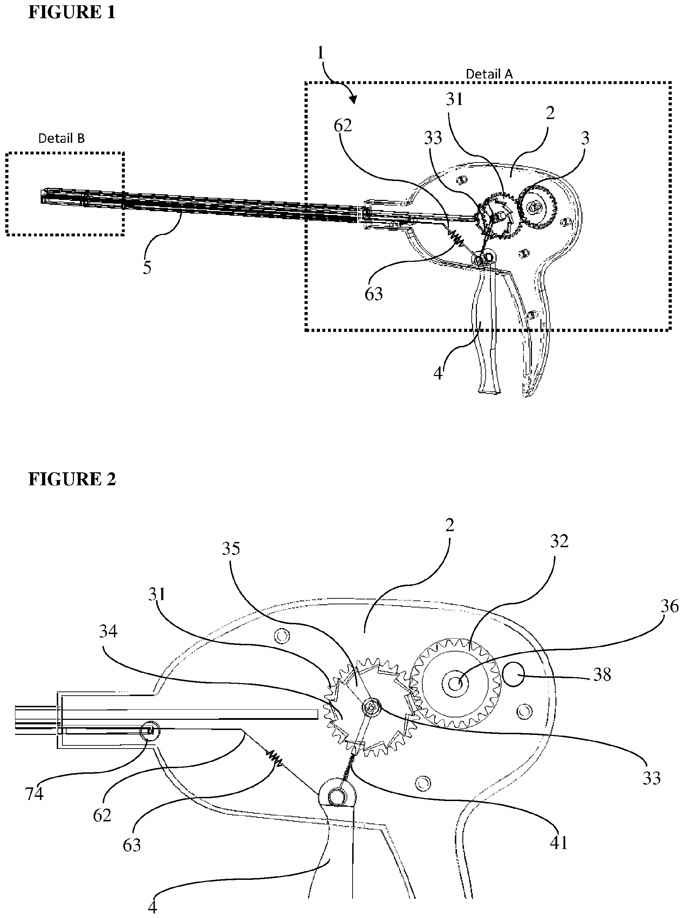

**Figure 1****.** is a transparent perspective view of the continuous stapling instrument.
**Figure 2****.** is a transparent perspective view of detail A.

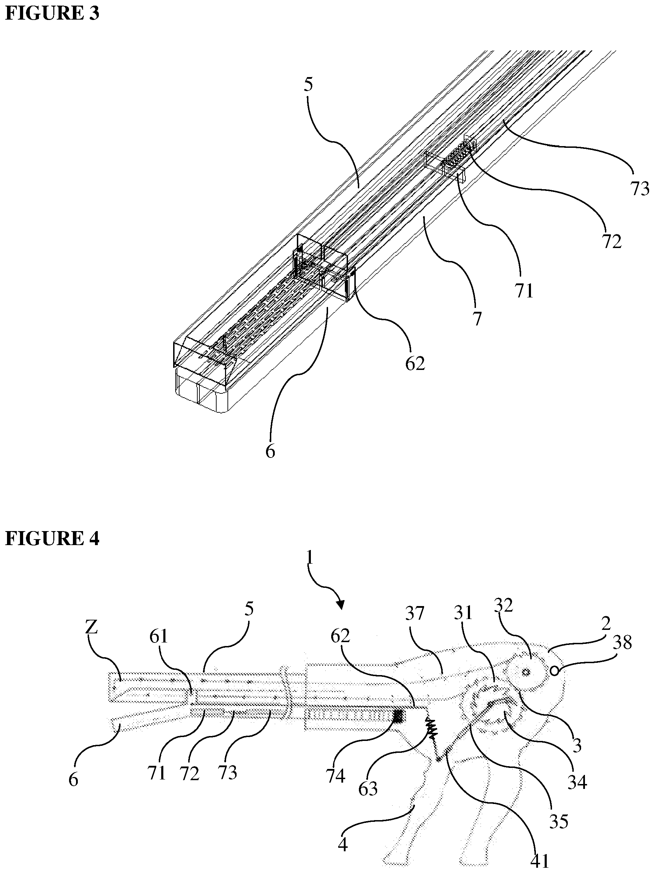

**Figure 3****.** is a transparent perspective view of detail B.
**Figure 4****.** is a transparent side view of the continuous stapling instrument.

The components in the figures are given reference numbers as follows:
**1.** Continuous stapling instrument
**2.** Body
**3.** Drive mechanism
   31. First gear
   32. Second gear
   33. First winding pulley
   34. Bevel helical gear
   35. Stopper Arm
   36. Second winding pulley
   37. Guide pin
   38. Winding pulley
**4.** Actuator rod
   41. Winding spring
**5.** Channel
**6.** Lower jaw
   61. Connection pin
   62. Wire rod
   63. Compression spring
**7.** Cutting channel
   71. Cutter blade
   72. Blade spring
   73. Blade shaft
   74. Blade pin
Z. Staple belt

A continuous stapling instrument (1), which performs stapling without being moved away from the area where the operation is being performed during stapling of the tissue in open, closed and/or robotic surgical operations, and which realizes winding of the emptied staple belt (Z), essentially comprises
- at least one body (2),
- at least one drive mechanism (3) which is located inside the body (2),
   - at least one first gear (31) which is located inside the body (2) and which can move circularly around the point at which it is connected,
   - at least one second gear (32) which is located inside body (2), can move circularly around the point at which it is connected, and contacts the first gear (31),
   - at least one first winding pulley (33), on which the full staple belt (Z) is wound, and which is attached to the center of the first gear (31) and moves coaxially with the first gear (31),
   - at least one bevel helical gear (34) which is attached to the end of the first winding pulley (33) and is positioned such that it is coaxial with the first winding pulley (33),
   - at least one stopper arm (34) which is coaxial with the bevel helical gear (34) and which can freely move on the central axis and extends towards the teeth of the bevel helical gear (34),
   - at least one second winding pulley (36), on which the empty staple belt (Z) is wound, and which is attached to the center of the second gear (32) and moves coaxially with the second gear (32),
   - at least one guide pin (37) which enables the staple belt (Z) to move straight and which is positioned on the inner surface of the body (2),
   - at least one winding pulley (38), which contacts the second gear (32), is mounted on the body (2) and extends outwardly from the body (2), and can rotate around its own axis,
- at least one actuator rod (4) which transfers the power required to operate the drive mechanism (3) and which is connected to the body (2),
   - at least one compression spring (41), whose one end is connected to the point where the actuator rod (4) is connected to the body (2) and the other end to the stopper arm (35), and which moves upon the movement of the actuator rod (4),
- at least one channel (5) which is connected onto the body (2) and in which the staple belt (Z) moves,
- at least one lower jaw (6) which is placed on the channel (5) and which is opened and closed serving as a jaw,
   - at least one connection pin (61), which enables to connect the lower jaw (6) onto the channel (5) and enables the lower jaw (6) to move around the point where it is connected,
   - at least one wire rod (62), whose one end is connected to the lower jaw (6) and the other end to the actuator rod (4), and which moves the lower jaw (6) upon movement of the actuator rod,
   - at least one compression spring (63) which is connected between the wire rod (62) and the actuator rod (4),
- at least one cutting channel (7) which is placed on the channel (5) and which enables to cut (transect) the tissue,
   - at least one cutter blade (71) which is located inside the cutting channel (7) placed on the channel (5) and enables to cut the tissue, and which can move linearly along the cutting channel (7),
   - at least one blade spring (72) to which the cutter blade (71) is connected,
   - at least one blade shaft (73) which is connected to the end of the blade spring (72) and which can move linearly along the cutting channel (7),
   - at least one blade pin (73) which is positioned on the blade shaft (73) and which enables the cutter blade (71) to be manually moved by the user.

In the present invention, the continuous stapling instrument (1), which performs stapling without being moved away from the area where the operation is being performed during stapling of the tissue in open, closed and/or robotic surgical operations, and which realizes winding of the emptied staple belt (Z), comprises a body (2) consisting of a hollow chamber. The corners of the body (2) are rounded to achieve a good appearance and ease of gripping and to enable it to be easily moveable and rotatable within the port in laparoscopic surgeries.

A drive mechanism (3) is located in the body (2). The drive mechanism (3) basically comprises a fist gear (31), a second gear (32), a first winding pulley (33), a bevel helical gear (34), a stopper arm (35), a second winding pulley (36) and a guide pin (37). A first gear (31), which is located inside body (2), can move circularly around the point at which it is connected. There is provided a second gear (32), which is located inside body (2), can move circularly around the point at which it is connected, and contacts the first gear (31). Upon circular movement of the first gear (31), the second gear (32), which is designed to mesh with the teeth of the first gear (31), also moves circularly. Thus, the first gear (31) transfers the circular movement to the second gear (32). The first winding pulley (33), on which the full staple belt (Z) is wound, and which is attached to the center of the first gear (31) and moves coaxially with the first gear (31). In a preferred embodiment of the present invention, the flat surface of the first winding pulley (33) having a flat cylinder form is mounted to the center of the first gear (31) and thus can move coaxially with the first gear (31). A bevel helical gear (34) is attached to the end of the first winding pulley (33) and the bevel helical gear (34) is positioned such that it is coaxial with the first winding pulley (33). The stopper arm (35) is coaxial with the bevel helical gear (34) and can move on its central axis. Furthermore, the stopper arm (35) extends towards the teeth of the bevel helical gear (34) and fits in between the teeth. Upon circular movement of the bevel helical gear (34), it moves together with the bevel helical gear (34); however, due to the tooth structure of the bevel helical gear (34), later on in the movement, it is gets off from the tooth and gets placed on another tooth. There is provided a second winding pulley (36), which is attached to the center of the second gear (32) on which the empty staple belt (Z) is wound, and which moves coaxially with the second gear (32), A guide pin (37) enables the staple belt (Z) to move linearly and is positioned on the inner surface of the body (2). In a preferred embodiment of the invention, there is provided a winding pulley (38) which contacts the second gear (32) and can rotate around its own central axis. The winding pulley (38) is mounted onto the body (2) and extends outwards from the body (2). Thanks to the fact that the winding pulley (38) extends outwards from the body (2), the user is enabled to rotate the winding pulley (38). The winding pulley (38) enables the user to intervene in the case that the staple belt (Z) gets entangled in the stapler instrument (1).

There is provided an actuator rod (4) which transfers the power required to operate the drive mechanism (3) and which is connected to the body (2). The actuator rod (4), which is connected onto the body (2) by the help of a connecting member, can move circularly around the point at which it is connected. There is provided a compression spring (41), whose one end is connected to the point where the actuator rod (4) is connected to the body (2) and the other end to the stopper arm (35), and which moves upon the movement of the actuator rod (4).

A channel (5) is provided which is connected onto the body (2) and in which the staple belt (Z) moves. A lower jaw (6) is placed on the channel (5), and the lower jaw (6) is opened and closed to compress the tissue and thus to support stapling of the staples in the staple belt (Z). Additionally, the lower jaw (6) helps the cutter blade (71) to fit into the cutting channel (7). In a preferred embodiment of the invention, there is a separating region within the channel (5) and the staple belt (Z) surrounds the said separating region. The separating region divides the channel (5) into two parts, namely staple belt (Z) inlet and staple belt (Z) outlet. The staple belt (Z), which enters through the staple belt (Z) inlet and is full of staples, moves by means of the drive mechanism (3) and proceeds up to the lower jaw (6). The staples on the staple belt (Z) are placed on the desired surface upon opening and closing of the lower jaw (6). In other words, upon opening and closing of the lower jaw (6), the tissue is compressed between the lower jaw (6) and the channel (5) and the staple on the staple belt (Z) penetrates the tissue and is closed in the form of a B shape. The staple belt (Z) emptied in the channel (5) is directed towards the staple belt (Z) outlet formed by the separating region and is wound on the second winding pulley (36). The lower jaw (6) is connected onto the channel (5) by means of a connection pin (61) and the connection pin (61) enables the lower jaw (6) to move around the point where it is connected. This way, the lower jaw (6) is opened and closed, and the staple in the full staple belt (Z) is placed on the desired surface. A wire rod (62), whose one end is connected to the lower jaw (6) and the other end to the actuator rod (4), is provided for moving the lower jaw (6) around the point where it is connected to the connection pin (61) upon movement of the actuator rod (4). A compression spring (63) is connected between the wire rod (62) and the actuator rod (4).

In a preferred embodiment of the invention, a cutting channel (7) is placed on the channel (5) and the cutting channel (7) enables the tissue to be cut (transected) and separated between the staples following completion of the stapling. A cutter blade (71) is located inside the cutting channel (7) that is placed on the channel (5), and the cutter blade (71) enables to cut the tissue and can move linearly along the cutting channel (7). The cutter blade (71) is connected to a blade spring (72). A blade shaft (73) is connected to the end of the blade spring (72) and it can move linearly along the cutting channel (7). There is provided a blade pin (74) positioned on the blade shaft (73). The blade pin (74) allows the cutter blade (71) to be moved manually by the user. The lower jaw (6) must be in closed position in order for the cutter blade (71) to perform cutting.

In the continuous stapling instrument (1) of the present invention, a part of the strip-shaped staple belt (Z) containing staples thereon is wound on the first winding pulley (33) and the other part extends within the channel (5). The staple belt (Z) emptied in the channel (5) leaves the channel (5) and is wound on the second winding pulley (36).

When the user pulls the actuator rod (4), the actuator rod (4) moves circularly around the point where it is connected to. There is provided a compression spring (41), whose one end is connected to the point where the actuator rod (4) is connected to the body (2) and the other end to the stopper arm (35), and which moves upon the movement of the actuator rod (4). The stopper arm (35), which extends towards the teeth of the bevel helical gear (34) that is connected to the compression spring (41) connected to the actuator rod (4), moves around the connection point at which the actuator rod (4) is connected. Upon movement of the stopper arm (35), the stopper arm (35) moves the bevel helical gear (34) circularly around the point where it is connected to. However, due to the compression spring (41), the stopper arm (35) does not move much with the bevel helical gear (34) and returns to the initial position. Upon circular movement of the bevel helical gear (34), the first winding pulley (33) and the first gear (31), which are connected to the bevel helical gear (34), move circularly. By means of the said movement, the full staple belt (Z) wound on the first winding pulley (33) enters into the channel (5) through the staple belt (Z) inlet provided in the channel (5). At the same time, upon circular movement of the first gear (31), the first gear (31) transfers the circular movement to the second gear (32). Upon circular movement of the second gear (32), the empty staple belt (Z) leaves through the staple belt (Z) outlet located on the channel (5) and is enabled to be wound on the second winding pulley (36) that is connected to the second gear (32).

Simultaneously with the movement of the staple belt (Z) resulting when the user pulls the actuator rod (4), the wire rod (62), which is connected to the point to which the actuator rod (4) is connected, moves linearly within the channel (5) and moves the lower jaw (6) around the point where it is connected to the connection pin (61). This way, upon opening and closing of the lower jaw (6), firstly, the tissue is compressed between the lower jaw (3) and the channel (5). When the user pulls the actuator rod (4) slightly further in order to staple the full staple belt (Z) coming from the channel (5) into the tissue, the compression spring (63) further compresses the tissue between the lower jaw (6) and the channel (5), and the staple on the staple belt (Z) is enabled to penetrate the tissue and to be formed into a B shape. This way, the staple on the full staple belt (Z) is placed on the desired surface.

When the user desires to cut (transect) the tissue during stapling, s/he brings the lower jaw (6) to closed position. Following that, the blade pin (74) located on the blade shaft (73) is moved to enable the cutter blade (71) to get out of the channel (5). After completion of the cutting (transection) process, when the user leaves the blade pin (74), the blade spring (72) places the cutter blade (71) back into the channel. The continuous stapling instrument (1) enables to perform transection of organs or bowel anastomosis during the entire surgical operation without the need for removal and insertion of the staple cartridges during surgery. Additionally, a safer and shorter stapling process, which can be performed without the need for removing the instrument from within the patient in the operations, is realized by means of the continuous stapling instrument (1). In addition, the continuous stapling instrument (1) enables the user to perform the stapling process with less effort.

## Claims

1. A continuous stapling instrument (1), which is configured to perform stapling without being moved away from the area where the operation is being performed during stapling of the tissue in open, closed and/or robotic surgical operations, and which is configured to realize winding of the emptied staple belt (Z), comprising:
- at least one body (2),
- at least one drive mechanism (3) which is located inside the body (2),
- at least one first gear (31) which is located inside the body (2) and which is configured to move circularly around the point at which it is connected,
- at least one second gear (32) which is located inside body (2), is configured to move circularly around the point at which it is connected, and contacts the first gear (31),
- at least one first winding pulley (33), on which the full staple belt (Z) is wound, and which is attached to the center of the first gear (31) and is configured to move coaxially with the first gear (31),
- at least one bevel helical gear (34) which is attached to the end of the first winding pulley (33) and is positioned such that it is coaxial with the first winding pulley (33),
- at least one stopper arm (34) which is coaxial with the bevel helical gear (34) and which is configured to freely move on the central axis and extends towards the teeth of the bevel helical gear (34),
- at least one second winding pulley (36), on which the empty staple belt (Z) is wound, and which is attached to the center of the second gear (32) and is configured to move coaxially with the second gear (32),
- at least one guide pin (37) which enables the staple belt (Z) to move straight and which is positioned on the inner surface of the body (2),
- at least one actuator rod (4) which is configured to transfer the power required to operate the drive mechanism (3) and which is connected to the body (2),
- at least one compression spring (41), whose one end is connected to the point where the actuator rod (4) is connected to the body (2) and the other end to the stopper arm (35), and which is configured to move upon the movement of the actuator rod (4),
- at least one channel (5) which is connected onto the body (2) and in which the staple belt (Z) is configured to move,
- at least one lower jaw (6) which is placed on the channel (5) and which is configured to open and close serving as a jaw,
- at least one connection pin (61), which enables to connect the lower jaw (6) onto the channel (5) and enables the lower jaw (6) to move around the point where it is connected,
- at least one wire rod (62), whose one end is connected to the lower jaw (6) and the other end to the actuator rod (4), and which is configured to move the lower jaw (6) upon movement of the actuator rod,
- at least one compression spring (63) which is connected between the wire rod (62) and the actuator rod (4).

2. A continuous stapling instrument (1) according to Claim 1, **comprising** at least one cutting channel (7) which is placed on the channel (5) and which enables to cut (transect) the tissue.

3. A continuous stapling instrument (1) according to Claim 2, **comprising** at least one cutter blade (71) which is located inside the cutting channel (7) placed on the channel (5) and enables to cut the tissue, and which is configured to move linearly along the cutting channel (7).

4. A continuous stapling instrument (1) according to Claim 3, **comprising** at least one blade spring (72) to which the cutter blade (71) is connected.

5. A continuous stapling instrument (1) according to Claim 4, **comprising** at least one blade shaft (73) which is connected to the end of the blade spring (72) and which is configured to move linearly along the cutting channel (7).

6. A continuous stapling instrument (1) according to Claim 4, **comprising** at least one blade pin (73) which is positioned on the blade shaft (73) and which enables the cutter blade (71) to be manually moved by the user.

7. A continuous stapling instrument (1) according to Claim 1, **comprising** a first winding pulley (33), which has a flat cylinder form, and whose flat surface is mounted to the center of the first gear (31), and which thus is configured to move coaxially with the first gear (31).

8. A continuous stapling instrument (1) according to Claim 1, **comprising** a second gear (32), which is designed to mesh with the teeth of the first gear (31).

9. A continuous stapling instrument (1) according to Claim 1, **comprising** a separating region which is positioned inside the channel (5) and which divides the channel (5) into two parts, namely the staple belt (Z) inlet and the staple belt (Z) outlet.

10. A continuous stapling instrument (1) according to Claim 1, **comprising** at least one winding pulley (38), which contacts the second gear (32), is mounted on the body (2) and extends outwardly from the body (2), and is configured to rotate around its own axis.

## Patentansprüche

1. Kontinuierliches Klammerinstrument (1), das konfiguriert ist, eine Klammerung durchzuführen, ohne dass es während der Klammerung des Gewebes bei offenen, geschlossenen und/oder robotergestützten chirurgischen Operationen von dem Bereich, in dem die Operation durchgeführt wird, wegbewegt wird, und das konfiguriert ist, ein Aufwickeln des ausgeleerten Klammerbandes (Z) zu realizieren, umfassend:
- mindestens ein Gehäuse (2),
- mindestens einen Antriebsmechanismus (3), der im Inneren des Gehäuses (2) angeordnet ist,
- mindestens ein erstes Zahnrad (31), das im Inneren des Gehäuses (2) angeordnet und konfiguriert ist, sich kreisförmig um den Punkt zu bewegen, an dem es angeschlossen ist,
- mindestens ein zweites Zahnrad (32), das im Inneren des Gehäuses (2) angeordnet und konfiguriert ist, sich kreisförmig um den Punkt zu bewegen, an dem es angeschlossen ist und das erste Zahnrad (31) kontaktiert,
- mindestens eine erste Wickelrolle (33), auf der der volle Klammerriemen (Z) aufgewickelt ist und die an der Mitte des ersten Zahnrads (31) befestigt ist und konfiguriert ist, sich koaxial mit dem ersten Zahnrad (31) zu bewegen,
- mindestens ein Schrägzahnkegelrad (34), das am Ende der ersten Wickelrolle (33) befestigt und so positioniert ist, dass es koaxial mit der ersten Wickelrolle (33) verläuft,
- mindestens einen zum Schrägzahnkegelrad (34) koaxialen Anschlagarm (34), der konfiguriert ist, sich frei auf der Mittelachse zu bewegen und sich in Richtung der Verzahnung des Schrägzahnkegelrads (34) zu erstrecken,
- mindestens eine zweite Wickelrolle (36), auf die der leere Klammerriemen (Z) aufgewickelt wird und die an der Mitte des zweiten Zahnrads (32) befestigt und konfiguriert ist, sich koaxial mit dem zweiten Zahnrad (32) zu bewegen,
- mindestens einen Führungsstift (37), der eine geradlinige Bewegung des Klammerriemen (Z) ermöglicht und an der Innenseite des Gehäuses (2) angeordnet ist,
- mindestens eine Betätigungsstange (4), die konfiguriert ist, die zur Betätigung des Antriebsmechanismus (3) erforderliche Kraft zu übertragen, und die mit dem Gehäuse (2) verbunden ist,
- mindestens einen Klemmring (41), dessen eines Ende mit dem Punkt verbunden ist, an dem die Betätigungsstange (4) mit dem Gehäuse (2) verbunden ist, und dessen anderes Ende mit dem Anschlagarm (35) verbunden ist, und der konfiguriert ist, sich bei Bewegung der Betätigungsstange (4) zu bewegen,
- mindestens einen Kanal (5), der an dem Gehäuse (2) angeschlossen ist und in dem der Klammerriemen (Z) konfiguriert ist,
- mindestens eine untere Klemmbacke (6) zu bewegen, die auf dem Kanal (5) aufgesetzt ist und die als Backe dienend zum Öffnen und Schließen konfiguriert ist und
- mindestens einen Verbindungsstift (61), der es ermöglicht, die untere Klemmbacke (6) mit dem Kanal (5) zu verbinden und ermöglicht der unteren Klemmbacke (6), sich um den Punkt zu bewegen, an dem sie verbunden ist,
- mindestens einen Walzdraht (62), dessen eines Ende mit der unteren Klemmbacke (6) und anderes Ende mit der Betätigungsstange (4) verbunden ist und der konfiguriert ist, die untere Klemmbacke (6) bei Bewegung der Betätigungsstange zu bewegen,
- mindestens eine Druckfeder (63), die zwischen dem Walzdraht (62) und der Betätigungsstange (4) angeschlossen ist.

2. Kontinuierliches Klammerinstrument (1) nach Anspruch 1, **umfassend** mindestens einen Schneidkanal (7), der auf den Kanal (5) aufgesetzt ist und ein Durchschneiden (Transektieren) des Gewebes ermöglicht.

3. Kontinuierliches Klammerinstrument (1) nach Anspruch 2, **umfassend** mindestens eine Schneidklinge (71), die sich innerhalb des auf dem Kanal (5) angeordneten Schneidkanals (7) befindet, und ein Durchschneiden des Gewebes ermöglicht, und die konfiguriert ist, sich linear entlang des Schneidkanals (7) zu bewegen.

4. Kontinuierliches Klammerinstrument (1) nach Anspruch 3, **umfassend** mindestens eine Klingenfeder (72), mit der die Schneidklinge (71) verbunden ist.

5. Kontinuerliches Klammerinstrument (1) nach Anspruch 4, **umfassend** mindestens eine Klingenwelle (73), die mit dem Ende der Klingenfeder (72) verbunden ist und die konfiguriert ist, sich linear entlang des Schneidkanals (7) zu bewegen.

6. Kontinuerliches Klammerinstrument (1) nach Anspruch 4, **umfassend** mindestens einen Klingenstift (73), der auf der Klingenwelle (73) positioniert ist und der es ermöglicht, die Schneidklinge (71) manuell durch den Benutzer zu bewegen.

7. Kontinuierliches Klammerinstrument (1) nach Anspruch 1, **umfassend** eine erste Wickelrolle (33), die eine flache Zylinderform aufweist und dessen flache Oberfläche an der Mitte des ersten Zahnrads (31) angebracht ist und der also konfiguriert ist, sich koaxial mit dem ersten Zahnrad (31) zu bewegen.

8. Kontinuierliches Klammerinstrument (1) nach Anspruch 1, **umfassend** ein zweites Zahnrad (32), das zum Eingriff in die Verzahnung des ersten Zahnrades (31) ausgelegt ist.

9. Kontinuierliches Klammerinstrument (1) nach Anspruch 1, **umfassend** einen Trennbereich, der innerhalb des Kanals (5) angeordnet ist und den Kanal (5) in zwei Teile, zwar in den Einlass des Klammerbandes (Z) und den Auslass des Klammerbandes (Z) unterteilt.

10. Kontinuierliches Klammerinstrument (1) nach Anspruch 1, **umfassend** mindestens eine Wickelrolle (38), die mit dem zweiten Zahnrad (32) in Kontakt steht, am Gehäuse (2) angeordnet ist und sich vom Gehäuse (2) nach außen erstreckt und konfiguriert ist, sich um ihre eigene Achse zu drehen.

## Revendications

1. Un instrument d'agrafage continu (1) qui est configuré pour réaliser un agrafage sans être éloigné de la zone où l'opération est effectuée pendant l'agrafage du tissu lors d'opérations chirurgicales ouvertes, fermés et/ou robotiques, et qui est configuré pour faire un enroulement de la bande d'agrafes (Z) vidée, comprenant:
- au moins un corps (2),
- au moins un mécanisme d'entraînement (3) qui est situé à l'intérieur du corps (2),
- au moins une première roue dentée (31) qui est située à l'intérieur du corps (2) et qui est configurée pour se déplacer circulairement autour du point auquel elle est reliée,
- au moins une deuxième roue dentée (32) qui est située à l'intérieur du corps (2), est configurée pour se déplacer circulairement autour du point auquel elle est reliée, et est en contact avec la première roue dentée (31),
- au moins une première poulie d'enroulement (33) sur laquelle la bande d'agrafes (Z) complète est enroulée et qui est attachée au centre de la première roue dentée (31) et est configurée pour se déplacer coaxialement avec la première roue dentée (31),
- au moins une roue conique hélicoïdale (34) qui est attachée à l'extrémité de la première poulie d'enroulement (33) et est positionnée de telle manière qu'elle est coaxiale avec la première poulie d'enroulement (33),
- au moins un bras d'arrêt (34) qui est coaxial avec la roue conique hélicoïdale (34) et configuré pour se déplacer librement sur l'axe central et qui s'étend vers les dents de la roue conique hélicoïdale (34),
- au moins une deuxième poulie d'enroulement (36) sur laquelle la bande d'agrafes (Z) vidée est enroulée et qui est attachée au centre de la deuxième roue dentée (32) et est configurée pour se déplacer coaxialement avec la deuxième roue dentée (32),
- au moins une goupille de guidage (37) qui permet la bande d'agrafes (Z) de se déplacer droit et qui est positionnée sur la surface intérieure du corps (2),
- au moins une tige d'actionnement (4) qui est configurée pour transférer la puissance nécessaire pour actionner le mécanisme d'entraînement (3) et qui est reliée au corps (2).
- au moins un ressort de pression (41), dont une extrémité est reliée au point où la tige d'actionnement (4) est reliée au corps (2) et l'autre extrémité au bras d'arrêt (35) et qui est configuré pour se déplacer lors du déplacement de la tige d'actionnement (4),
- au moins un canal (5) qui est relié sur le corps (2) et dans lequel la bande d'agrafes (Z) est configurée pour se déplacer,
- au moins une mâchoire inférieure (6) qui est placée sur le canal (5) et qui est configurée pour s'ouvrir et se fermer servant d'une mâchoire,
- au moins une goupille de connexion (61), qui permet de relier la mâchoire inférieure (6) sur le canal (5) et permet à la mâchoire inférieure (6) de se déplacer autour du point où elle est reliée,
- au moins une tige de fil (62), dont une extrémité est reliée à la mâchoire inférieure (6) et l'autre extrémité à la tige d'actionnement (4), et qui est configurée pour déplacer la mâchoire inférieure (6) lors de déplacement de la tige d'actionnement,
- au moins un ressort de pression (63) qui est relié entre la tige de fil (62) et la tige d'actionnement (4).

2. Un instrument d'agrafage continu (1) selon la Revendication 1, **comprenant** au moins un canal coupant (7) qui est placé sur le canal (5) et qui permet de couper (trancher) le tissu.

3. Un instrument d'agrafage continu (1) selon la Revendication 2, **comprenant** au moins une lame coupante (71) qui est située à l'intérieur du canal coupant (7) placé sur le canal (5) et qui permet de couper le tissu, et qui est configurée pour se déplacer linéairement le long du canal coupant (7).

4. Un instrument d'agrafage continu (1) selon la Revendication 3, **comprenant** au moins un ressort de lame (72) auquel la lame coupante (71) est reliée.

5. Un instrument d'agrafage continu (1) selon la Revendication 4, **comprenant** au moins un arbre de lame (73) qui est relié à l'extrémité du ressort de lame (72) et qui est configuré pour se déplacer linéairement le long du canal coupant (7).

6. Un instrument d'agrafage continu (1) selon la Revendication 4, **comprenant** au moins un axe de lame (73) qui est positionné sur l'arbre de lame (73) et qui permet à la lame coupante (71) d'être manuellement déplacée par l'utilisateur.

7. Un instrument d'agrafage continu (1) selon la Revendication 1, **comprenant** au moins une première poulie d'enroulement (33), qui a une forme cylindrique plate, et dont la surface plate est montée au centre de la première roue dentée (31), et qui par conséquent est configurée pour se déplacer coaxialement avec la première rue dentée (31).

8. Un instrument d'agrafage continu (1) selon la Revendication 1, **comprenant** une deuxième roue dentée (32), qui est conçue pour se raccorder avec les dents de la première roue dentée (31).

9. Un instrument d'agrafage continu (1) selon la Revendication 1, **comprenant** une zone de séparation qui est positionnée à l'intérieur du canal (5) et qui sépare le canal (5) en deux parties, à savoir l'entrée de la bande d'agrafes (Z) et la sortie de la bande d'agrafes (Z) .

10. Un instrument d'agrafage continu (1) selon la Revendication 1, **comprenant** au moins une poulie d'enroulement (38), qui est en contact avec la deuxième roue dentée (32), qui est montée sur le corps (2) et qui s'étend vers l'extérieur à partir du corps (2), et est configurée pour tourner autour de son propre axe.
